Europäisches Patentamt

European Patent Office

Office européen des brevets

(11) Veröffentlichungsnummer: **0 180 887 B1**

# EUROPÄISCHE PATENTSCHRIFT

(45) Veröffentlichungstag der Patentschrift: **21.08.91**

(51) Int. Cl.⁵: **A61F 2/16**

(21) Anmeldenummer: **85113693.7**

(22) Anmeldetag: **28.10.85**

(54) Einstückige Hinterkammer Implantationslinse.

(30) Priorität: **29.10.84 DE 3439551**

(43) Veröffentlichungstag der Anmeldung:
**14.05.86 Patentblatt 86/20**

(45) Bekanntmachung des Hinweises auf die
Patenterteilung:
**21.08.91 Patentblatt 91/34**

(84) Benannte Vertragsstaaten:
**BE DE FR GB IT LU NL**

(56) Entgegenhaltungen:
**EP-A- 0 064 770**
**DE-B- 2 556 665**
**US-A- 4 162 122**

(73) Patentinhaber: **INPROHOLD Establishment**
**Schwefelstrasse 33**
**FL-9490 Vaduz(LI)**

(72) Erfinder: **Schlegel, Hans-Joachim, Prof. Dr.**
**med.**
**Siebenpfeifferstrasse 22**
**W-6650 Homburg/Saar(DE)**

(74) Vertreter: **Wey, Hans-Heinrich, Dipl.-Ing. et al**
**Patentanwälte Wey & Partner Widenmayer-**
**strasse 49**
**W-8000 München 22(DE)**

## Beschreibung

Die Erfindung betrifft eine einstückige Hinterkammer-Implantationslinse als Ersatz für die aus dem Auge von Lebewesen höherer Ordnung operativ, insbesondere extrakapsulär entfernte natürliche Linse.

Implantationslinsen der vorbezeichneten Art sind Gegenstand des Europäischen Patents 064 770 und sind somit aus der EP-A-0 064 770 bekanntgeworden. Sie haben sich bereits in der ophthalmologischen Praxis hervorragend bewährt. Diese Linsen besitzen einen zentralen, als Sammellinse ausgebildeten Linsenkörper mit sich von diesem peripher radial nach außen erstreckenden und diesen in seiner Lage fixierenden Halterungen in Form dünnwandiger flächiger Stützelemente, deren äußerer Rand auf einem Kreisbogen um den Mittelpunkt des Linsenkörpers liegt, der sich entweder im Linsenkapselsack oder in der Ziliarfurche abstützt, und welche aus einem homogenen, glasklaren, hochtemperaturbeständigen Kunststoff, vorzugsweise aus vulkanisiertem Silikonwerkstoff bestehen, welcher ein spezifisches Gewicht zwischen 0,01 und 1,08, vorzugsweise von etwa 1,02, besitzt.

Es ist der Wunsch geäußert worden, Linsen der in Betracht kommenden Art, welche aus einem flexiblen Werkstoff bestehen, falten zu können, um sie durch einen kleinstmöglichen Schnitt von nur wenigen Millimetern Länge in das Auge einbringen zu können, nachdem die natürliche getrübte Augenlinse extrakapsulär entfernt worden ist. Auf der einen Seite besteht die Forderung, daß der Werkstoff der Implantationslinse verhältnismäßig weich und flexible, aber doch steif genug sein soll, um eine Formstabilität der Linse zu gewährleisten, andererseits soll die Linse aber aus vorerwähnten Gründen faltbar sein. Die Faltbarkeit von Linsen mit einer entsprechenden Dicke des zentralen Linsenkörpers im Hinblick auf die notwendige Brechkraft ist jedoch begrenzt.

Der Erfindung liegt somit die Aufgabe zugrunde, eine Hinterkammer-Implantationslinse der fraglichen Art zu schaffen, die alle Vorteile und Eigenschaften der vorbekannten Linse aufweist, jedoch aufgrund einer speziellen Formgebung geeignet ist, gefaltet werden zu können, um ihre Dimension in Querrichtung zum Zwecke des Einbringens in das Auge durch einen minimalen Schnitt hindurch auf ein Mindestmaß zu verringern.

Um allen in Betracht kommenden Forderungen gerecht zu werden, wird zur Lösung der Aufgabe vorgeschlagen, die Hinterkammer-Implantationslinse in der Weise auszubilden, daß wenigstens eine der beiden Oberflächen des zentralen Linsenkörpers in der Art der optisch wirksamen Fläche einer Fresnel-Linse ausgebildet ist, d.h. daß die Linse aus einzelnen periaxialen Ringzonen zusammengesetzt ist, zwischen welchen sich Abstufungen befinden. Die Krümmungsradien der einzelnen Zonenbereiche sind so gewählt, daß die Brennpunkte aller Zonen zusammenfallen. Dadurch ist es möglich, die Linse relativ dünnwandig auszubilden, ihr Volumen erheblich zu reduzieren und so auch eine nennenswerte Gewichtsersparnis zu erreichen. Insbesondere aber lassen sich derart ausgebildete Linsen leichter falten, um ihre Breite in gefaltetem Zustand auf etwa die Hälfte der ursprünglichen Breite zu bringen.

Weitere Werkmale der erfindungsgemäß ausgebildeten Implantationslinsen gehen aus den Unteransprüchen sowie aus der nachstehenden Beschreibung einer Reihe bevorzugter Ausführungsbeispiele hervor, welche in den Figuren 1 bis 10 der Zeichnungen dargestellt sind. Es zeigen:

Fig. 1 bis 6     je einen Querschnitt durch eine gemäß der Erfindung ausgebildete Hinterkammer-Implantationslinse in unterschiedlicher Ausführungsform;

Fig. 7 bis 10     Aufsichten auf Hinterkammer-Implantationslinsen gemäß den Figuren 1 bis 6 mit unterschiedlichen Umrißformen.

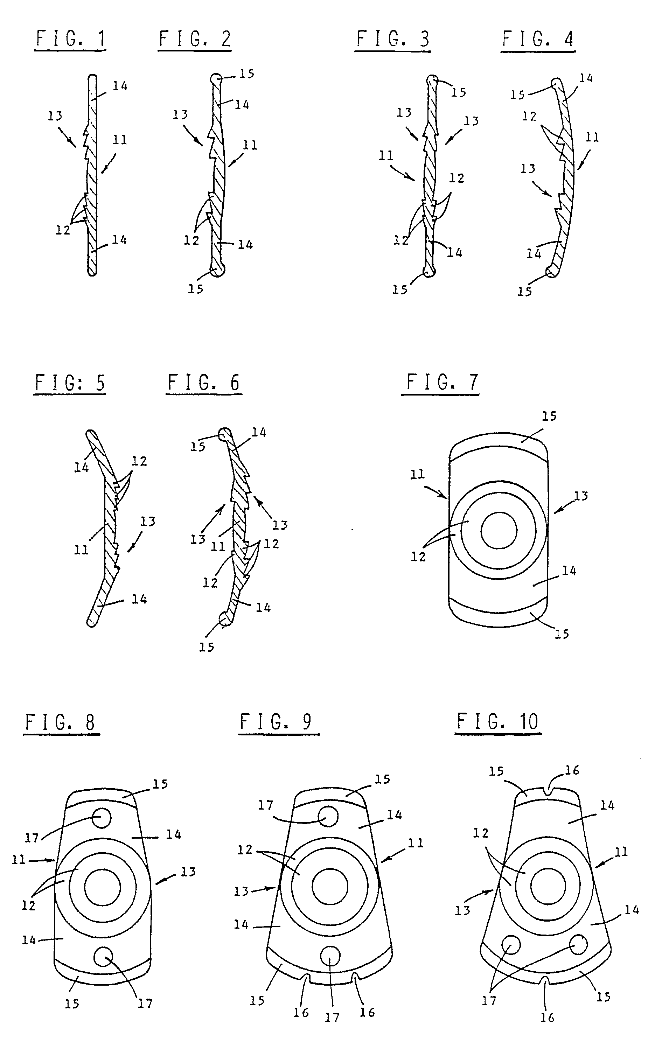

Die Hinterkammer-Implantationslinse nach Fig. 1 besteht aus einer ebenen Scheibe aus glasklarem flexiblem Werkstoff, in deren Mitte ein zentraler Linsenkörper 11 angeordnet ist; dieser besteht auf der Vorderseite aus den abgestuften Ringzonen 12 einer Fresnel-Linse 13. Die Rückseite des Linsenkörpers 11 ist z.B. plan ausgebildet. Die Linse besteht aus einem einzigen Stück. Die Stützelemente 14 sind an der Peripherie des zentralen Linsenkörpers angeordnet.

Die Linse nach Fig. 2 entspricht derjenigen nach Fig. 1. Bei ihr ist lediglich die Rückseite des zentralen Linsenkörpers 11 nach rückwärts gewölbt. Am äußeren Umfang der Stützelemente 14 befindet sich ein abgerundeter Ringwulst 15, um die Auflagefläche auf dem empfindlichen Gewebe zu vergrößern und die Flächenpressung zu verringern.

Bei der Linse nach Fig. 3 sind die beiden Oberflächen des zentralen Linsenkörpers 11 mit abgestuften Ringen 12 versehen. Die Linse nach Fig. 4 ist zur Rückseite hin über ihre ganze Höhe gewölbt. Die vordere Oberfläche des zentralen Linsenkörpers 11 besteht aus abgestuften Ringen 12 einer Fresnel-Linse.

Die Implantationslinse nach Fig. 5 besitzt einen zentralen Linsenkörper 11 aus einer plankonvexen Linse, deren rückwärtige konvexe Oberfläche abgestuft ist. Die Stützelemente 14 sind gegenüber dem zentralen Linsenkörper 11 nach vorn abgewinkelt. Die Ausführungsform der Implantationslinse nach Fig. 6 entspricht im wesentlichen derjenigen nach Fig. 5. Der zentrale bikonvexe Linsenkörper 11 ist in diesem Falle sowohl auf der Vorderseite als auch auf der Rückseite abgestuft. Die Stützelemente 14 sind wiederum mit einem äußeren peripheren Ringwulst 15 versehen.

Bei den Ausführungsformen der Hinterkammer-Implantationslinse nach den Figuren 5 und 6 ergibt sich der Vorteil, daß sich die noch im Auge stehende Hinterkapsel nicht unmittelbar an der zentralen Rückfläche der Implantationslinse anlegen kann, weil sie durch deren nach hinten vorspringende Stufen in einem erwünschten geringen Abstand von dieser gehalten wird. Der dadurch gebildete Zwischenraum ist wichtig für den Fall, daß sich auf der Hinterkapsel Nachstarbildungen entwickeln, die dann mit Hilfe eines Yag-Lasers zerstört werden können, ohne gleichzeitig auch die Implantationslinse zu beschädigen.

In den Figuren 7 bis 10 sind Ausführungsformen für die Hinterkammer-Implantationslinse dargestellt, deren zentrale Linsenkörper in der in den Figuren 1 bis 6 dargestellten Weise mit Stufen 12 versehen sein können.

Implantationslinsen, bei welchen der untere Rand des unteren Stützelements 14 mit einem Ringwulst 15 ausgestattet ist und bei welchen der untere Rand breiter ist als der Durchmesser des zentralen Linsenkörpers, haben den Vorteil, daß die Berührungsfläche, an welcher das Gewebe anliegt, größer ist als die Berührungsfläche am oberen Rand, so daß die häufig zu beobachtende Erscheinung, daß sich die Implantationslinse im Auge dreht, vermeidbar ist. Gegebenenfalls können auch in den Wulsten 15 eine oder zwei Kerben 16 angeordnet sein, durch welche der Tendenz der Linse, sich zu drehen, entgegengewirkt wird.

Alle zuvor beschriebenen und dargestellten Linsen bieten den Vorteil, außerordentlich leicht und flach zu sein, so daß sie auch, wenn gewünscht, faltbar sind, um sie durch einen kleinstmöglichen Schnitt in der Hornhaut nach Entfernung der getrübten Linse durch diesen hindurch in die Vorderkammer oder gegebenenfalls auch in die Hinterkammer einbringen zu können.

Wenn die Abstufung der Linsenringe 12 derart gewählt wird, daß die einzelnen Ringzonen der Fresnel'schen Stufenlinse unter dem Auflösungsvermögen des Auges liegen, werden sie nicht mehr als Ringe bemerkt und wirken folglich in der gleichen Weise wie eine Linse mit durchgehend gekrümmter Oberfläche.

Es ist von Vorteil, in den Stützelementen 14 Öffnungen 17 anzubringen, durch die Augenkammerwasser hindurchtreten kann und die im Falle des Faltens der Linse dies begünstigen.

**Patentansprüche**

1. Einstückige Hinterkammer-Implantationslinse als Ersatz für die aus dem Auge von Lebewesen höherer Ordnung operativ, insbesondere extrakapsulär entfernte natürliche Linse, welche

    a) einen zentralen, als Sammellinse ausgebildeten Linsenkörper (11), und

    b) an dem Linsenkörper angeordnete, sich von diesem peripher radial nach außen erstreckende und diesen in seiner Lage fixierende Halterungen (14,15) in Form dünnwandiger flächiger Stützelemente, deren äußerer Rand auf einem Kreisbogen um den Mittelpunkt des Linsenkörpers liegt, und welcher sich entweder im Linsenkapselsack oder in der Ziliarfurche abstützt, aufweist,

und welche aus einem homogenen, glasklaren, hochtemperaturbeständigen Kunststoff, vorzugsweise aus vulkanisiertem Silikonwerkstoff, besteht, welcher ein spezifisches Gewicht zwischen 1,01 und 1,08, vorzugsweise von etwa 1,02, besitzt, dadurch gekennzeichnet, daß wenigstens eine der beiden Oberflächen des zentralen Linsenkörpers (11) in der Art der optisch wirksamen Fläche einer Fresnel-Linse (13) ausgebildet ist.

2. Implantationslinse nach Anspruch 1, dadurch gekennzeichnet, daß die vordere Fläche des zentralen Linsenkörpers (11) in der Art der optisch wirksamen Fläche einer Fresnel-Linse (13) ausgebildet ist.

3. Implantationslinse nach Anspruch 1 oder 2, dadurch gekennzeichnet, daß die hintere Fläche des Linsenkörpers (11) als gewölbte Fläche ausgebildet ist.

4. Implantationslinse nach Anspruch 2 oder 3, dadurch gekennzeichnet, daß die Mittenebene der Linsen gewölbt ist.

5. Implantationslinse nach einem der Ansprüche 1 bis 4, dadurch gekennzeichnet, daß die Stützelemente (14) gegenüber dem Linsenkörper (11) geneigt sind.

6. Implantationslinse nach einem oder mehreren der Ansprüche 1 bis 5, dadurch gekennzeichnet, daß das untere Stützelement (14) eine breitere periphere Auflage- und Stützfläche aufweist als das obere Stützelement (14).

7. Implantationslinse nach einem oder mehreren der Ansprüche 1 bis 6, dadurch gekennzeichnet, daß wenigstens das untere Stützelement (14) mit einem Wulstrand (15) versehen ist.

8. Implantationslinse nach Anspruch 7, dadurch gekennzeichnet, daß im Wulstrand (15) wenigstens eine Kerbe (16) oder dgl. angeordnet ist.

9. Implantationslinse nach einem oder mehreren der Ansprüche 1 bis 8, dadurch gekennzeichnet, daß die Stützelemente (14) mit wenigstens einer Öffnung (17) versehen sind.

## Claims

1. A single posterior chamber implantable lens as a replacement for the natural lens surgically removed, in particular extracapsularly, from the eye of living beings of a higher order, having
   a) a central lens body (11) provided as a collective lens and
   b) holders (14, 15) arranged on the lens body, extending radially outwards from the periphery of the body and fixing it in position, provided in the form of thin-walled, tabular support elements with the outer edges lying on an arc around the center point of the lens body and supported either in the lens capsule sack or in the ciliary groove,
   and consisting of a homogenous, crystal clear plastic resistant to high teperatures, preferably a vulcanized silicone material with a specific gravity of between 1.01 and 1.08, preferably approximately 1.02,
   characterized in that
   at least one of the two surfaces of the central lens body (11) is provided as the optically effective surface of a Fresnel lens (13).

2. an implantable lens according to claim 1, characterized in that the front surface of the central lens body (11) is provided as the optically effective surface of a Fresnel lens (13).

3. An implantable lens according to claim 1 or 2, characterized in that the rear surface of the lens body (11) is provided as a curved surface.

4. An implantable lens according to claim 2 or 3, characterized in that the central area of the lenses is curved.

5. An implantable lens according to claims 1 to 4, characterized in that the support elements (14) are inclined relative to the lens body (11).

6. An implantable lens according to one or more of claims 1 to 5, characterized in that the lower support element (14) has a broader peripheral bearing and supporting surface than the upper support element (14).

7. An implantable lens according to one or more of claims 1 to 6, characterized in that at least the lower support element (14) is provided with a bead edge (15).

8. An implantable lens according to claim 7, characterized in that at least one notch (16) or the like is located in the bead edge (15).

9. An implantable lens according to one or more of claims 1 to 8, characterized in that the support elements (14) are provided with at least one hole (17).

## Revendications

1. Lentille d'implantation intraoculaire de chambre postérieure, composée d'une seule pièce et devant remplacer la lentille naturelle extraite par opération, notamment extracapsulaire, de l'oeil d'êtres vivants supérieurs, lentille d'implantation qui
   a) possède un corps lenticulaire central (11) en forme de lentille convergente
   b) possède des éléments de fixation disposés sur le corps lenticulaire (14,15) allant de la périphérie de ce corps de façon radiale vers l'extérieur et maintenant le corps lenticulaire dans sa position, fixations sous forme d'éléments d'appui plans et d'épaisseur faible et dont le bord extérieur s'appuie en arc de cercle autour du centre du corps lenticulaire, ce bord extérieur s'appuyant soit sur le sac capsulaire soit dans le sillon ciliaire
   et qui est constituée d'une matière synthétique homogéne limpide résistant à des températures élevées, préférentiellement d'un matériau siliconé vulcanisé d'un poids spécifique entre 1,01 et 1,08, préférentiellement d'environ 1,02, caractérisée par le fait qu'au moins une des deux surfaces du corps lenticulaire central (11) forme une surface optiquement active d'une lentille de Fresnel (13).

2. Lentille d'implantation selon revendication 1), caractérisée par le fait que la face antérieure du corps lenticulaire central (11) possède une surface optiquement active d'une lentille de Fresnel (13).

3. Lentille d'implantation selon revendications 1) ou 2) caractérisée par le fait que la face postérieure du corps lenticulaire (11) forme une surface courbe.

4. Lentille d'implantation selon revendication 2) ou 3) caractérisée par le fait que la face centrale des lentilles est courbée.

5. Lentille d'implantation selon l'une des revendications 1) à 4) caractérisée par le fait que les éléments d'appuis (14) sont inclinés par rapport au corps lenticulaire (11).

6. Lentille d'implantation selon l'une ou plusieurs des revendications 1) à 5) caractérisée par le fait que l'élément d'appui inférieur (14) possède une surface de contact et d'appui périphérique plus large que l'élemént d'appui supérieur (14).

7. Lentille d'implantation selon une ou plusieurs des revendications 1) à 6) caractérisée par le fait qu'au moins l'élément d'appui inférieur (14) est pouvu d'un bourrelet (15).

8. Lentille d'implantation selon revendication 7) caractérisée par le fait que le bourrelet (15) comporte au moins une encoche (16) ou similaire.

9. Lentille d'implantation selon une ou plusieurs des revendications 1) à 8) caractérisée par le fait que les éléments d'appui (14) sont pourvus d'au moins une ouverture (17).

FIG. 1    FIG. 2    FIG. 3    FIG. 4

FIG: 5    FIG. 6    FIG. 7

FIG. 8    FIG. 9    FIG. 10